# EUROPÄISCHE PATENTSCHRIFT

(11) **EP 1 154 777 B1**
(45) Veröffentlichungstag und Bekanntmachung des Hinweises auf die Patenterteilung: **11.05.2005**
(21) Anmeldenummer: 00903681.5
(22) Anmeldetag: 10.02.2000
(51) Int. Cl.: A61K 31/55, A61P 9/12

(54) **ARZNEIMITTEL ZUR BEHANDLUNG VON BLUTHOCHDRUCK**
MEDICAMENT FOR TREATING HYPERTENSION
MEDICAMENT POUR LE TRAITEMENT DE L'HYPERTENSION ARTERIELLE

(30) Priorität: 16.02.1999 DE 19906310
(43) Veröffentlichungstag der Anmeldung: 21.11.2001
(73) Patentinhaber: Solvay Pharmaceuticals GmbH, 30173 Hannover (DE)
(72) Erfinder: WILKINS, Martin, R., Buckinghamshire SL2 4DF (GB); THORMÄHLEN, Dirk, D-31039 Rheden (DE); WALDECK, Harald, D-30916 Isernhagen (DE)
(74) Vertreter: Gosmann, Martin, Dr.
(86) Internationale Anmeldenummer: PCT/EP2000/001068
(87) Internationale Veröffentlichungsnummer: WO 2000/048601

(56) Entgegenhaltungen:
- DE-A- 19 510 566
- DE-A- 19 638 020
- GB-A- 2 207 351
- US-A- 4 749 688
- US-A- 5 362 727

## Beschreibung

Die vorliegende Erfindung betrifft die Verwendung von Benzazepin-N-essigsäurederivaten, welche in α-Stellung zu dem Stickstoffatom eine Oxogruppe enthalten und in 3-Stellung durch einen 1-(Carboxyalkyl)-cyclopentyl-carbonyl-amino-Rest substituiert sind, und deren Salzen und biolabilen Estern zur Behandlung von Bluthochdruck, insbesondere von bestimmten Formen des sekundären Bluthochdrucks, in größeren Säugetieren und insbesondere Menschen, und zur Herstellung von für diese Behandlung geeigneten Arzneimitteln. Die Ursache des zu behandelnden Bluthochdrucks kann hierbei auf verschiedenster Genese beruhen. Insbesondere betrifft die Erfindung die Behandlung von solchen Formen des sekundären Bluthochdrucks, die infolge verschiedener nicht-kardialer Krankheiten auftreten können.

Benzazepin-N-essigsäurederivate, welche in α-Stellung zu dem stickstoffatom eine Oxogruppe enthalten und in 3-Stellung durch einen 1-(Carboxyalkyl)-cyclopentyl-carbonyl-amino-Rest substituiert sind, und deren Salze und biolabilen Ester fallen unter den Schutzumfang von in der deutschen Patentanmeldung DE 195 10 566 beschriebenen Benzazepin-, Benzoxazepin- und Benzothiazepin-N-essigsäurederivaten, welche in α-Stellung zu dem Stickstoff eine Oxogruppe enthalten und in 3-Stellung durch einen 1-(Carboxyalkyl)-cyclopentylcarbonyl-amino-Rest substituiert sind, und NEP-inhibitorische Wirkungen am Herzen besitzen. Die hier im Rahmen der vorliegenden Erfindung verwendeten Benzazepin-N-essigsäure-Verbindungen können nach den in der DE 195 10 566 beschriebenen Verfahren hergestellt werden.

Der Erfindung liegt die Aufgabe zugrunde, neue pharmazeutische Zubereitungen zur Behandlung von Bluthochdruck, insbesondere zur Behandlung von von bestimmten Formen des sekundären Bluthochdrucks zu entwickeln. Bevorzugt betrifft die Aufgabe der Erfindung die Entwicklung neuer pharmazeutischer Zubereitungen zur Behandlung von solchen Formen des sekundären Bluthochdrucks, die infolge verschiedener nicht-kardialer Krankheiten auftreten können.

Erfindungsgemäß werden nun Verbindungen der allgemeinen Formel I 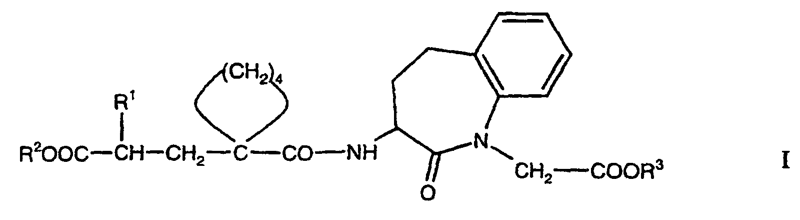 worin
- R¹: für eine Phenylniederalkylgruppe, welche gegebenenfalls im Phenylring durch niederes Alkyl, niederes Alkoxy oder Halogen substituiert sein kann, oder für eine Naphthylniederalkylgruppe steht,
- R²: Wasserstoff oder eine einen biolabilen Ester bildende Gruppe bedeutet und
- R³: Wasserstoff oder eine einen biolabilen Ester bildende Gruppe bedeutet
und physiologisch verträgliche Salze der Säuren der Formel I verwendet zur Herstellung von pharmazeutischen Zubereitungen zur Behandlung von Bluthochdruck, insbesondere zur Behandlung von bestimmten Formen des sekundären Bluthochdrucks, in größeren Säugetieren und Menschen.

Sofern in den Verbindungen der Formel I die Substituenten niedere Alkyl- oder Alkoxygruppen bedeuten oder enthalten, können diese geradkettig oder verzweigt sein und insbesondere 1 bis 4, vorzugsweise 1 bis 2, Kohlenstoffatome enthalten und stellen bevorzugt Methyl oder Methoxy dar. Sofern die Substituenten Halogen enthalten, kommen insbesondere Fluor, Chlor oder Brom, vorzugsweise Fluor oder Chlor in Frage.

In dem Rest R¹ kann die Niederalkylenkette 1 bis 4, vorzugsweise 1 bis 2 Kohlenstoffatome enthalten. Insbesondere stellt R¹ eine gegebenenfalls substituierte Phenethylgruppe dar, welche gegebenenfalls ein- oder mehrfach durch Halogen, niederes Alkoxy oder niederes Alkyl substituiert sein kann, oder eine Naphthylethylgruppe.

Die Verbindungen der Formel I stellen gegebenenfalls veresterte Dicarbonsäurederivate dar. Je nach Applikationsform sind biolabile Monoester, insbesondere Verbindungen, worin R² eine einen biolabilen Ester bildende Gruppe und R³ Wasserstoff bedeuten, oder Dicarbonsäuren bevorzugt, wobei letztere insbesondere für i.v.-Applikation geeignet sind.

Als biolabile Ester bildende Gruppen R² und R³ eignen sich niedere Alkylgruppen, gegebenenfalls im Phenylring durch niederes Alkyl oder durch eine an zwei benachbarte Kohlenstoffatome gebundene niedere Alkylenkette substituierte Phenyl- oder Phenylniederalkylgruppen, im Dioxolanring gegebenenfalls durch niederes Alkyl substituierte Dioxolanylmethylgruppen oder gegebenenfalls an der Oxymethylgruppe durch niederes Alkyl substituierte C₂-C₆-Alkanoyloxymethylgruppen. Sofern die einen biolabilen Ester bildende Gruppe R² oder R³ niederes Alkyl bedeutet, kann dieses eine bevorzugt unverzweigte Alkylgruppe mit 1 bis 4, vorzugsweise 2 Kohlenstoffatomen darstellen. Sofern die einen biolabilen Ester bildende Gruppe eine gegebenenfalls substituierte Phenylniederalkylgruppe darstellt, kann deren Alkylenkette 1 bis 3, vorzugsweise 1, Kohlenstoffatome enthalten. Sofern der Phenylring durch eine niedere Alkylenkette substituiert ist, kann diese 3 bis 4, insbesondere 3 Kohlenstoffatome enthalten. Als phenylhaltige Substituenten R² und/oder R³ eignen sich insbesondere Phenyl, Benzyl oder Indanyl. Sofern R² und/oder R³ eine gegebenenfalls substituierte Alkanoyloxymethylgruppe darstellen, kann deren Alkanoyloxygruppe 2 bis 6, vorzugsweise 3 bis 5, Kohlenstoffatome enthalten und ist vorzugsweise verzweigt und kann beispielsweise einen Pivaloyloxymethylrest (= tert.-Butylcarbonyloxymethylrest) darstellen.

Als physiologisch verträgliche Salze von Dicarbonsäuren oder Monoestern der Formel I kommen deren Alkalimetall-, Erdalkalimetall- oder Ammoniumsalze in Frage, beispielsweise Natrium- oder Kalziumsalze oder Salze mit physiologisch verträglichen, pharmakologisch neutralen organischen Aminen wie beispielsweise Diethylamin oder tert.-Butylamin.

Die Verbindungen der Formel I enthalten zwei chirale Kohlenstoffatome, nämlich das die Amidseitenkette tragende Kohlenstoffatom in 3-Stellung des Ringgerüstes und das den Rest R¹ tragende Kohlenstoffatom der Amidseitenkette. Die Verbindungen können somit in mehreren optisch aktiven stereoisomeren Formen oder als Racemat vorliegen. Gemäß der vorliegenden Erfindung können sowohl die racemischen Gemische als auch die isomerenreinen Verbindungen der Formel I verwendet werden.

Es wurde nun überraschenderweise gefunden, daß die erfindungsgemäß verwendete Gruppe der Verbindungen der Formel I - insbesondere auch im Hinblick auf bestimmte sekundäre Formen des Bluthochddrucks - eine blutdrucksenkende Wirkung am Menschen und größeren Säugetieren besitzen. Die Verbindungen der Formel I und deren physiologisch verträglichen Salze der Säuren und deren biolabile Ester eignen sich somit zur Behandlung von Bluthochdruck, insbesondere zur Behandlung von bestimmten Formen des sekundären Bluthochdrucks, wobei der zu behandelnde Bluthochdruck verschiedenster Genese sein kann. Die Verbindungen der Formel I, einschließlich deren Salze von Säuren und deren biolabile Ester, eignen sich hierbei vorteilhaft zur Behandlung von solchen Formen des sekundären Bluthochdrucks, die infolge verschiedener nicht-kardialer Krankheiten auftreten können.

Unter Bluthochdruck (Hypertonie) versteht man eine Erhöhung des Blutdrucks über die Norm hinaus, die vor allem als arterielle Hypertonie in Erscheinung tritt. Mit Blick auf die Entstehungsursache des Bluthochdrucks werden zwei grundsätzliche Formen, der essentielle oder primäre Bluthochdruck einerseits und die Formen des sekundären Bluthochdrucks andererseits, unterschieden. Essentieller Bluthochdruck ist in der Regel bedingt durch erhöhten Strömungswiderstand infolge anfangs rein funktioneller, später organischer Engstellung der arteriellen Blutbahn. Der sekundäre oder auch symptomatische Bluthochdruck ist dagegen ein organgebundener, d.h. durch die Erkrankung eines Organs ausgelöster Bluthochdruck, der sich u.a. z.B. als endokrine, renale, pulmonale oder kardiovaskuläre Hypertonie äußern kann. Die ursächlich für den sekundären Bluthochdruck verantwortlichen Erkrankungen können vielfältiger Art sein, z.B. chronisch obstruktive Lungen- und Atemwegserkrankungen oder chronisches Asthma. So findet die normale Blutzirkulation in der Lunge beim erwachsenen Menschen bei niederem Druck und geringem Widerstand statt. Eine bestehende chronische Hypoxie, wie diese z.B. bei chronisch obstruktiven Atemwegserkrankungen auftreten kann, führt jedoch zu pulmonaler arterieller Hypertension und zum Remodelling der Lungenarteriolen (verstärktes Wachstum von Gefäßmuskelzellen) und des rechten Ventrikels (verstärktes Wachstum von Herzmuskelzellen).

Besonders vorteilhaft lassen sich die Verbindungen der Formel I, einschließlich deren Salze von Säuren und deren biolabile Ester zur Behandlung von pulmonalem Bluthochdruck, insbesondere auch bei nicht-kardialem Ursprung, einsetzen. Pulmonaler Bluthochdruck kann hierbei als primäre Form (mit unbekannter Ursache) oder als sekundärer pulmonaler Bluthochdruck vorliegen und mit den Verbindungen der Formel I und deren physiologisch verträglichen Salzen der Säuren und deren biolabile Ester behandelt werden.

Unter (sekundärem) pulmonalem Bluthochdruck (Hochdruck im kleinen Kreislauf) versteht man eine konstante Mitteldruckerhöhung im Lungenarteriensystem auf Werte > 22 mmHg in Ruhe. Diese Mitteldruckerhöhung kann z.B. auftreten infolge herzbedingter Stauung im kleinen Kreislauf (z.B. Mitralvitien, Linksherzinsuffizienz), Gefäßverkrampfung vor dem Kapillargebiet (z.B. infolge Hypoxie bei Höhenaufenthalt, obstruktivem Lungenemphysem, nach lungenverkleinernden Operationen), sekundären Gefäßschwundes (bei Lungenfibrose, destruktivem Lungenemphysem), Überdurchblutung, d.h. Hyperzirkulation im Lungenkreislauf mit nachfolgender lichtungseinengender Gefäßerkrankung (z.B. bei Herzfehler mit großem Links-rechts-Shunt), rezidivierender Lungenembolien, als Nebenwirkung bei Einnahme bestimmter Appetitzügler (z.B. Aminorex) oder auch infolge primärer Lungengefäßverengungen (= idiopathischer = primär vaskulärer pulmonaler Hochdruck).

Zur erfindungsgemäßen Behandlung des Bluthochdrucks können die Verbindungen der Formel I und deren physiologisch verträglichen Salze der Säuren und deren biolabile Ester in üblichen pharmazeutischen Zubereitungen oral, intravenös oder auch transdermal verabreicht werden.

So können die Verbindungen der Formel I und deren physiologisch verträglichen Salze der Säuren und deren biolabile Ester in einer blutdrucksenkend wirksamen Menge zusammen mit üblichen pharmazeutischen Hilfs- und/oder Trägerstoffen in festen oder flüssigen pharmazeutischen Zubereitungen enthalten sein. Als Beispiele fester Präparate seien oral applizierbare Präparate wie Tabletten, Dragees, Kapseln, Pulver oder Granulate genannt, oder auch Supporitorien oder Pflaster (transdermale therapeutische Systeme). Diese festen Präparate können pharmazeutisch übliche anorganische und/oder organische Trägerstoffe wie z.B. Milchzucker, Talkum oder Stärke neben pharmazeutisch üblichen Hilfsmitteln, beispielsweise Gleitmitteln oder Tabletten-sprengmitteln, enthalten. Flüssige Präparate wie Lösungen, Suspensionen oder Emulsionen der Wirkstoffe können die üblichen Verdünnungsmittel wie Wasser, Öle und/oder Suspensionsmittel wie Polyethylenglykole und dergleichen enthalten. Es können zusätzlich weitere Hilfsstoffe zugegeben werden, wie z.B. Konservierungsmittel, Geschmackskorrigenzien und dergleichen.

Die Wirkstoffe können mit den pharmazeutischen Hilfs- und/oder Trägerstoffen in an sich bekannter Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Das Granulat oder Pulver kann direkt in Kapseln abgefüllt oder in üblicher Weise zu Tablettenkernen verpreßt werden. Diese können gewünschtenfalls in bekannter Weise dragiert werden. Flüssige Zubereitungen können durch Auflösen oder Dispergieren der Wirkstoffe und ggf. weiterer Hilfsstoffe in einem geeigneten flüssigen Träger in Form von Lösungen oder Suspensionen erhalten werden.

Die blutdrucksenkende Wirkung der erfindungsgemäß verwendeten Verbindungen der Formel I kann in pharmakologischen Tests in vivo an chronisch hypoxischen Ratten durch Messung der Substanzwirkung in Bezug auf hierfür geeignete pharmakologische Indikatoren, z.B. durch Messung des pulmonalen Arteriendrucks und des rechten Ventrikelgewichtes, sowie durch Untersuchung des pulmonalen Gefäß-Remodellings in hypoxischen Ratten nachgewiesen werden.

### Beschreibung der Testmethoden und Ergebnisse

Als Testsubstanz wurde stellvertretend für die erfindungsgemäß verwendbaren Substanzen der Formel I (3S,2R')-3-[1-(2'-(Carboxy-4'-phenyl-butyl)-cyclopentan-1-carbonylamino]-2,3,4,5-tetrahydro-2-oxo-1H-1-benzazepin-1-essigsäure eingesetzt. Die verabreichte Dosis der Substanz betrug 40 mg/kg pro Tag (ausreichend, um die Druck-Rückkopplung auf Big Endothelin zu unterdrücken).

Als Versuchstiere wurden Sprague-Dawley Ratten (260 bis 310 g; n = 6 bis 10 pro Versuchsgruppe) verwendet. Zur Erzeugung von pulmonalem Bluthochdruck wurden die Tiere in einer Druckkammer akut hypoxischen Bedingungen ausgesetzt. Eine Kontrollgruppe wurde zum Vergleich unter normaler Luft und eine weitere Kontrollgruppe unter hypoxischen Bedingungen gehalten.

### Die Tests wurden wie folgt durchgeführt:

Die Versuchstiere wurden mit der aktiven Substanz oder einem Vehikel mittels osmotischer Minipumpen behandelt. Die osmotischen Minipumpen wurden den Tieren intraperitonal implantiert, bevor die Tiere 24 h später für einen Zeitraum von 2 Wochen in einer Druckkammer untergebracht wurden. Dort wurden die Tiere bei Normaldruck entweder unter hypoxischen Bedingungen (10 % O₂) oder bei normaler Luft gehalten. Nach 2 Wochen wurden die Tiere für die haemodynamischen Untersuchungen vorbereitet und die Messungen durchgeführt.

### Haemodynamische Untersuchungen:

Die Tiere wurden betäubt und danach in üblicher Weise eine vorgefertigte Kanüle über die rechte Jugular-Vene, Vorhof und Herzkammer in die Lungenarterie eingeführt. Ein Katheter wurde in die linke Jugular-Vene eingesetzt, um Wirkstoffe intravenös verabreichen zu können. Die linke Karotis-Arterie wurde zur Messung des systemischen Blutdrucks ebenfalls mit einer Kanüle versehen. Nach Aufwachen der Tiere wurde der pulmonale Arteriendruck (PAP = pulmonary artery pressure) aufgezeichnet. Dann wurden die Ratten für 10 Minuten in einer Minikammer erneut hypoxischen Bedingungen (10 % O₂) ausgesetzt und der Anstieg des pulmonalen Arteriendrucks gemessen und ebenfalls aufgezeichnet. Die Effekte der Testsubstanz auf den PAP unter normoxischen und hypoxischen Bedingungen sind in Tabelle 1 im Vergleich zu Kontrollversuchen dargestellt. Die angegebenen Daten sind Mittelwerte ± Standardabweichungen und wurden mittels ANOVA statistisch ausgewertet.

### Messung des antihypertrophen Effektes:

Nach Beendigung der haemodynamischen Untersuchungen wurden die Tiere getötet und die Herzen herauspräpariert. Die Gewichte der rechten und linken Herzkammern wurden bestimmt und in Relation zum Körpergewicht gesetzt. Die Effekte der Testsubstanz auf die Herzgewichte unter normoxischen und hypoxischen Bedingungen sind in Tabelle 1 im Vergleich zu Kontrollversuchen dargestellt. Die angegebenen Daten sind Mittelwerte ± Standardabweichungen und wurden mittels ANOVA statistisch ausgewertet.

### Untersuchung des Effektes auf das pulmonal-arterielle Remodelling:

Nach Tötung der Ratten wurden neben den Herzen auch die Lungen isoliert. Letztere wurden histologisch untersucht, d.h. das Ausmaß der distalen Lungengefäßmuskularisierung wurde nach Anfärbung nach "van Gieson" durch Mikroskopie bei 400-facher vergrößerung bestimmt. Die Effekte der Testsubstanz auf das pulmonal-arterielle Remodelling (d.h. auf die Muskularisierung der distalen Lungengefäße) während einer 2-wöchigen Hypoxie sind in Tabelle 2 im Vergleich zu hypoxischen Kontrollversuchen dargestellt. Die angegebenen Daten sind Mittelwerte ± Standardabweichungen und wurden mittels ANOVA statistisch ausgewertet.

### Ergebnisse:

Bei der beschriebenen Testmethode führte die Behandlung mit der Testsubstanz bei hypoxischen Tieren zu einer statistisch signifikanten Erniedrigung des pulmonalen Arteriendrucks (PAP), verglichen zu den hypoxischen Kontrolltieren (Tabelle 1). Dabei wurde der normale systemische Blutdruck nicht beeinflußt, d.h. es wurden keine hypotensiven Eigenschaften festgestellt. Dies stellt einen besonderen Vorteil dar, da bei normotensiven Personen mit pulmonalem Bluthochdruck kein Blutdruckabfall unter Normalwerte zu befürchten ist.

**Tabelle 1:**

| Effekte der erfindungsgemäß verwendeten Substanz (40 mg/kg/Tag über 14 Tage) auf den pulmonalen Arteriendruck und die Rechts- und Linksherzgewichte von Ratten unter 14-tägigen normoxischen und hypoxischen Bedingungen. | | | | |
|---|---|---|---|---|
| Parameter | Normoxie | | Hypoxie | |
| | Kontrolle n=9 | Testsubstanz n=8 | Kontrolle n=10 | Testsubstanz n=9 |
| PAP (mmHg) | 19,9 ± 2 | 22,2 ± 1 | 42,9 ± 1,6* | 33,2 ± 1,2*# |
| RtHWt (mg) | 178 ± 10 | 171 ± 10 | 269 ± 4,5* | 242 ± 6,4*# |
| Rt/LtHWt (mg/mg) | 0,25 ± 0,01 | 0,25 ± 0,01 | 0,45 ± 0,01* | 0,40 ± 0,02* |
| RtHWt/BW (mg/g) | 0,54 ± 0,02 | 0,56 ± 0,03 | 0,94 ± 0,02* | 0,87 ± 0,03* |

| | | | | |
|---|---|---|---|---|
| In der Tabelle bedeuten: * signifikant verschieden im Vergleich zu normoxischen Kontrollversuchen (p<0,05) | | | | |
| # signifikant verschieden im Vergleich zu hypoxischen Kontrollversuchen (p<0,05) PAP = pulmonary artery pressure = pulmonaler Arteriendruck RtHWt = Gewicht der rechten Herzkammer (mg); Rt/LtHWt Verhältnis des Gewichts der rechten zur linken Herzkammer; RtHWt/BW = Verhältnis des Gewichts der rechten Herzkammer zum Körpergewicht. | | | | |

Die Verminderung des pulmonalen Arteriendrucks durch die Testsubstanz führte zu einer statistisch signifikanten Reduktion des Rechtsherz-Gewichtes der Ratten (antihypertropher Effekt) im Vergleich zu hypoxischen Kontrollversuchen (Tabelle 1). Es war auch eine Tendenz zur Reduktion der Gewichtsverhältnisse des rechten zum linken Herzgewicht und des rechten Herzgewichtes zum Körpergewicht zu verzeichnen (Tabelle 1).

Weiterhin reduzierte die Testsubstanz statistisch signifikant die Muskularisierung der distalen Lungengefäße der Ratten (Tabelle 2). Dieses verminderte pulmonal-arterielle Remodelling ist ebenfalls eine Folge der statistisch signifikanten Reduktion des pulmonalen Bluthochdruckes.

**Tabelle 2:**

| Effekte der erfindungsgemäß verwendeten Substanz (40 mg/kg/Tag über 14 Tage) auf die Muskularisierung distaler Lungengefäße von Ratten während 14-tägiger Hypoxie im Vergleich zu hypoxischen Kontrollversuchen | | |
|---|---|---|
| Parameter | 14 Tage Hypoxie | |
| | Kontrolle n=6 | Testsubstanz n=8 |
| Muskularisiert (%) | 76 ± 4 | . 52 ± 5* |
| Partiell muskularisiert (%) | 23 ± 4 | 39 ± 4* |
| Nicht-muskularisiert (%) | 1 ± 1 | 9 ± 3 |

| | | |
|---|---|---|
| * signifikant verschieden im Vergleich zu hypoxischen Kontrollversuchen (p<0,05) | | |

Aufgrund ihrer vorstehend beschriebenen Wirkung sind die Verbindungen der Formel I und deren Salze und biolabilen Ester geeignet als Arzneimittel für größere Säugetiere und Menschen zur Behandlung von Bluthochdruck, insbesondere zur Behandlung von bestimmten Formen des sekundären Bluthochdrucks. Die erfindungsgemäß verwendeten Verbindungen eignen sich hierbei besonders zur Behandlung von solchen Formen des sekundären Bluthochdrucks, die infolge verschiedener nicht-kardialer Krankheiten auftreten können, wie vorzugsweise z.B. zur Behandlung von nicht-kardial bedingtem pulmonalen Bluthochdruck. Die erfindungsgemäß verwendeten Substanzen bieten somit einen vorteilhaften Ansatz zur Behandlung und/oder Prophylaxe von insbesondere Hypoxie-bedingtem pulmonalen Bluthochdruck und dessen Komplikationen, jedoch ohne den normalen systemischen Blutdruck zu beeinträchtigen.

Hierbei werden Dicarbonsäuren der Formel I und deren Salze zweckmäßig in parenteral, insbesondere i.v., applizierbaren Arzneiformen und Mono- oder Diester der Formel I zweckmäßig in oral applizierbaren Arzneiformen eingesetzt. Die zu verwendenden Dosen können individuell verschieden sein und variieren naturgemäß je nach Art des zu behandelnden Zustandes, der verwendeten Substanz und der Applikationsform. Zum Beispiel werden parenterale Formulierungen im allgemeinen weniger Wirkstoff enthalten als orale Präparate. Im allgemeinen eignen sich jedoch für Applikationen an größeren Säugetieren, insbesondere Menschen, Arzneiformen mit einem Wirkstoffgehalt von 1 bis 200 mg pro Einzeldosis. Die Verbindungen der Formel I, einschließlich deren Salze von Säuren und deren biolabile Ester können hierbei in pharmazeutischen Zubereitungen sowohl zur sofortigen als auch verzögerten, kontrollierten und/oder gesteuerten Wirkstoff-Freisetzung verabreicht werden.

Die nachfolgenden Beispiele sollen die Erfindung näher erläutern, jedoch deren Umfang in keiner weise beschränken.

Die nachfolgenden Beispiele 1 und 2 beschreiben erfindungsgemäße pharmazeutische Zubereitungen enthaltend einen Wirkstoff der Formel I sowie die Herstellung solcher pharmazeutischer Zubereitungen. Die erfindungsgemäß verwendeten Verbindungen der Formel I können hierfür nach den in der bereits genannten deutschen Patentanmeldung DE 195 10 566 beschriebenen Methoden hergestellt werden. Beispiel 3 nennt bevorzugte Verbindungen für die erfindungsgemäße Verwendung.

### Beispiel 1:

### (3S,2'R)-3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure enthaltende Tabletten

Man stellte Tabletten in folgender Zusammensetzung pro Tablette her:

| | |
|---|---|
| (3S,2'R)-3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure | 20 mg |
| Maisstärke | 60 mg |
| Milchzucker | 135 mg |
| Gelatine (als 10%ige Lösung) | 6 mg |

Der Wirkstoff, die Maisstärke und der Milchzucker wurden mit der 10%igen Gelatine-Lösung eingedickt. Die Paste wurde zerkleinert, und das entstehende Granulat wurde auf ein geeignetes Blech gebracht und bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

### Beispiel 2:

### (3S,2'R)-3-[1-(2'-Carboxy-4'-phenyl-butyl)-cyclopentan-1-carbonylamino]-2,3,4,5-tetrahydro-2-oxo-1H-1-benzazepin-1-essigsäure enthaltende Injektionslösung.

Man stellte eine Injektionslösung mit folgender Zusammensetzung pro 5 ml her:

| | |
|---|---|
| (3S,2'R)-3-[1-(2'-Carboxy-4'-phenyl-butyl)-cyclopentan-1-carbonylamino]-2,3,4,5-tetrahydro-2-oxo-1H-1-benzazepin-1-essigsäure | 10 mg |
| Na₂HPO₄·7H₂O | 43,24 mg |
| NaH₂PO₄·2H₂O | 7,72 mg |
| NaCl | 30,0 mg |
| gereinigtes Wasser | 4948,0 mg |

Die Feststoffe wurden in Wasser gelöst, die Lösung wurde sterilisiert und in Portionen von jeweils 5 ml in Ampullen abgefüllt.

### Beispiel 3:

Bevorzugte Verbindungen der Formel I für die erfindungsgemäße Verwendung zur Herstellung von Arzneimitteln zur Behandlung von Bluthochdruck, insbesondere zur Behandlung von sekundären Formen des Bluthochdrucks wie z.B. pulmonaler Bluthochdruck, sind z.B. (einschließlich der Salze von Säuren):
3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure-tert.-butylester.
3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure.
(3S,2'R)-3-{1-[2'-Ethoxycarbonyl)-4'-phenyl-butyl]-cyclopentan-1-carbonylamino}-2,3,4,5-tetrahydro-2-oxo-1H-1-benzazepin-1-essigsäure-tert.-butylester.
(3S,2'R)-3-{1-[2'-(Ethoxycarbonyl)-4'-phenyl-butyl]-cyclopentan-1-carbonylamino}-2,3,4,5-tetrahydro-2-oxo-1H-1-benzazepin-1-essigsäure.
(35.2'R)-3-{1-[2'-(Carboxy-4'-phenyl-butyl]-cydopentan-1-carbomylamino}-2,3,4,5-tetrahydro-2-oxo-1H-1-benzazepin-1-essigsäure.
3-{1-[2'-(tert.-Butoxycarbonyl)-4'-phenyl-butyl)-cyclopentan-1-carbonylamino}-2,3,4,5-tetrahydro-2-oxo-1H-1-benzazepin-1-essigsäure-tert.butylester.
3-[1-(2'-Carboxy-4'-phenyl-butyl)-cyclopentan-1-carbonylamino]-2,3,4,5-tetrahydro-2-oxo-1H-1-benzazepin-1-essigsäure.
3-{1-[2'-(tert.-Butoxycarbonyl)-4'-phenyl-butyl]-cyclopentan-1-carbonylamino}-2,3,4,5-tetrahydro-2-oxo-1H-1-benzazepin-1-essigsäure-benzylester.
3-[1-(2'-Carboxy-4'-phenyl-butyl)-cyclopentan-1-carbonylamino]-2,3,4,5-tetrahydro-2-oxo-1H-1-benzazepin-1-essigsäure-benzylester.
3-{1-[2'-(tert.-Butylcarbonyloxymethoxycarbonyl)-4'-phenylbutyl]-cyclopentan-1-carbonylamino}-2,3,4,5-tetrahydro-2-oxo-1H-1-benzazepin-1-essigsäure-benzylester.
3-{1-[2'-(Pivaloyloxymethoxycarbonyl)-4'-phenyl-butyl]-cyclopentan-1-carbonylamino}-2,3,4,5-tetrahydro-2-oxo-1H-1-benzazepin-1-essigsäure.

## Patentansprüche

1. Verwendung von Verbindungen der allgemeinen Formel I 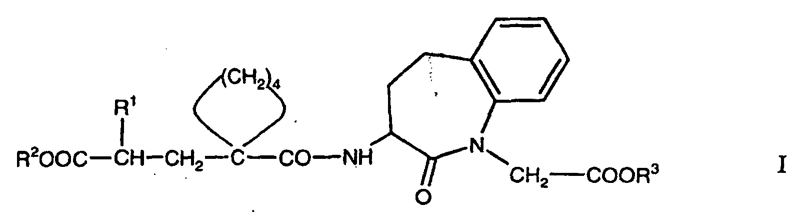 worin
R¹ für eine Phenyl-(C1- bis C4-alkyl)gruppe, welche gegebenenfalls im Phenylring durch C1- bis C4-Alkyl, C1- bis C4-Alkoxy oder Halogen substituiert sein kann, oder für eine Naphthyl-(C1- bis C4-alkyl)gruppe steht,
R² Wasserstoff oder eine einen biolabilen Ester bildende Gruppe bedeutet und
R³ Wasserstoff oder eine einen biolabilen Ester bildende Gruppe bedeutet,
worin die einen biolabilen Ester bildende Gruppe in R² und/oder R³ eine C1- bis C4-Alkylgruppe darstellt, eine gegebenenfalls im Phenylring durch C1- bis C4-Alkyl oder durch eine an 2 benachbarte Kohlenstoffatome gebundene C1- bis C4-Alkylenkette substituierte Phenyl- oder Phenyl-(C1- bis C4-alkyl)gruppe, insbesondere Phenyl, Benzyl oder Indanyl, darstellt, eine im Dioxolanring gegebenfalls durch C1- bis C4-Alkyl substituierte Dioxolanylmethylgruppe, insbesondere (2,2-Dimethyl-1,3-dioxolan-4-yl)methyl, darstellt, oder eine gegebenenfalls an der Oxymethylgruppe durch C1- bis C4-Alkyl substituierte C₂-C₆-Alkanoyloxymethylgruppe darstellt.
und physiologisch verträglichen Salzen der Säuren der Formel I zur Herstellung von pharmazeutischen Zubereitungen zur Behandlung von sekundären Formen des Bluthochdrucks, insbesondere von solchen Formen des sekundären Bluthochdrucks, die durch nicht-kardiale Erkrankungen bedingt sind, in größeren Säugetieren und Menschen.

2. Verwendung von Verbindungen gemäß Anspruch 1, **dadurch gekennzeichnet, daß** der sekundäre, durch nicht-kardiale Erkrankungen bedingte Bluthochdruck ein pulmonaler Bluthochdruck ist.

3. Verwendung von Verbindungen gemäß Anspruch 1 oder 2, worin R² und/oder R³ eine einen biolabilen Ester bildende Gruppe wie in Anspruch 1 definiert bedeuten.

4. Verwendung von Verbindungen nach einem der vorstehenden Ansprüche **dadurch gekennzeichnet, daß** R² eine einen biolabilen Ester bildende Gruppe in Anspruch 1 definiert bedeutet und R³ Wasserstoff ist.

5. Verwendung von Verbindungen gemäß Anspruch 4, **dadurch gekennzeichnet, daß** (3S,2'R)-3-{1-[2'-(Ethoxycarbonyl)-4'phenyl-butyl]-cyclopentan-1-carbonylamino}-2,3,4,5-tetrahydro-2-oxo-1H-1-benzazepin-1-essigsäure oder deren physiologisch verträgliche Salze eingesetzt werden.

## Claims

1. Use of compounds of the general formula I 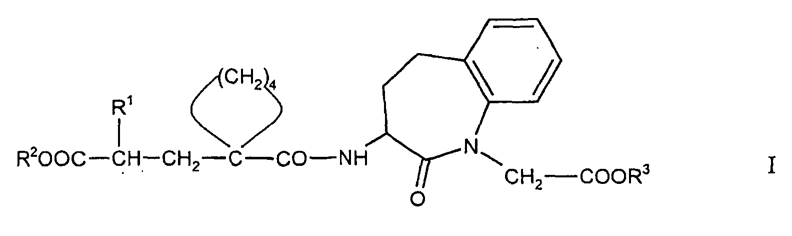 where
R¹ stands for a phenyl-(C1 to C4-alkyl) group which can optionally be substituted in the phenyl ring by C1 to C4 alkyl, C1 to C4 alkoxy or halogen, or for a naphthyl-(C1 to C4-alkyl) group,
R² means hydrogen or a group forming a biolabile ester and
R³ means hydrogen or a group forming a biolabile ester,
in which the group forming a biolabile ester in R² and/or R³ is a C1 to C4 alkyl group, or a phenyl or phenyl-(C1 to C4-alkyl) group, particularly phenyl, benzyl or indanyl, which is optionally substituted in the phenyl ring by C1 to C4 alkyl or by a C1 to C4 alkylene chain bonded to two adjacent carbon atoms, or a dioxolanylmethyl group, particularly (2,2-dimethyl-1,3-dioxolan-4-yl)methyl, which is optionally substituted in the dioxolane ring by C1 to C4 alkyl, or a C₂-C₆-alkanoyloxymethyl group optionally substituted on the oxymethyl group by C1 to C4 alkyl, and physiologically acceptable salts of the acids of formula I for the production of pharmaceutical compositions for the treatment of secondary forms of hypertension, particularly of those forms of secondary hypertension which are caused by non-cardiac diseases, in larger mammals and humans.

2. Use of compounds according to claim 1, **characterised in that** the secondary hypertension caused by non-cardiac diseases is pulmonary hypertension.

3. Use of compounds according to claim 1 or 2, in which R² and/or R³ means a group forming a biolabile ester as defined in Claim 1.

4. Use of compounds according to one of the above claims, **characterised in that** R² is a group forming a biolabile ester as defined in Claim 1 and R³ is hydrogen.

5. Use of compounds according to claim 4, **characterised in that** (3S,2'R)-3-{1-[2'-(ethoxycarbonyl)-4'-phenylbutyl]-cyclopentane-1-carbonylamino}-2,3,4,5-tetrahydro-2-oxo-1H-1-benzazepine-1-acetic acid or physiologically acceptable salts thereof are used.

## Revendications

1. Utilisation de composés de la formule générale I 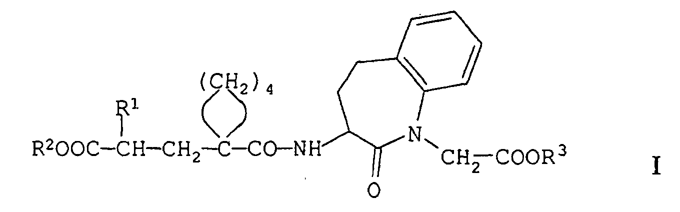 dans laquelle :
R¹ représente un groupe (phényl)alkyle en C₁ à C₄, qui peut être éventuellement substitué au niveau du noyau phényle par un alkyle en C₁ à C₄, un alcoxy en C₁ à C₄ ou un halogène, ou bien représente un groupe (naphtyl)alkyle en C₁ à C₄,
R² représente l'hydrogène ou un groupe formant un ester biolabile, et
R³ représente l'hydrogène ou un groupe formant un ester biolabile,
où le groupe formant un ester biolabile dans R² et/ou R³ représente un groupe alkyle en C₁ à C₄, un groupe phényle ou phényl(alkyle en C₁ à C₄) éventuellement substitué au niveau du noyau phényle par un alkyle en C₁ à C₄ ou par une chaîne alkylène en C₁ à C₄ liée à deux atomes de carbone voisins, en particulier un groupe phényle, benzyle ou indanyle, un groupe dioxolanylméthyle éventuellement substitué au niveau du noyau dioxolane par un alkyle en C₁ à C₄, en particulier un groupe (2,2-diméthyl-1,3-dioxolan-4-yl)méthyle, ou encore un groupe (alcanoyle en C₂ à C₆)oxyméthyle éventuellement substitué au niveau du groupe oxyméthyle par un alkyle en C₁ à C₄,
ainsi que les sels physiologiquement compatibles des acides de formule I, pour l'élaboration de préparations pharmaceutiques pour le traitement des formes de l'hypertension secondaire, en particulier des formes de l'hypertension secondaire qui sont provoquées par des maladies non cardiaques, chez les grands mammifères et l'homme.

2. Utilisation de composés selon la revendication 1, **caractérisée en ce que** l'hypertension secondaire provoquée par des maladies non cardiaques est une hypertension pulmonaire.

3. Utilisation de composés selon la revendication 1 ou la revendication 2, dans laquelle R² et/ou R³ représentent un groupe formant un ester biolabile tel que défini dans la revendication 1.

4. Utilisation de composés selon l'une des revendications précédentes, **caractérisée en ce que** R² représente un groupe formant un ester biolabile tel que défini dans la revendication 1 et R³ représente l'hydrogène.

5. Utilisation de composés selon la revendication 4, **caractérisée en ce que** l'on utilise de l'acide (3S,2'R)-3-{1-[2'-(éthoxycarbonyl)-4'-phénylbutyl]-cyclopentane-1-carbonylamino}-2,3,4,5-tétrahydro-2-oxo-1H-1-benzazépine-1-acétique ou ses sels physiologiquement compatibles.
